# EUROPEAN PATENT APPLICATION

(11) **EP 1 025 880 A1**
(43) Date of publication of application: **09.08.2000**
(21) Application number: 98938940.8
(22) Date of filing: 21.08.1998
(51) Int. Cl.: A63B 23/04, A63B 23/12

(54) **MUSCLE STRENGTHENING TOOL**

(71) Applicant: Kabushikikaisha Sato Sports Plaza, Fuchu-shi, Tokyo 183-0016 (JP)
(72) Inventor: SATO, Yoshiaki, Fuchu-shi, Tokyo 183-0016 (JP)
(74) Representative: Manitz, Finsterwald & Partner GbR
(86) International application number: JP9803721
(87) International publication number: WO0010649

(57) **Abstract**

A muscle potentiating implement is provided which is used for simply and safely performing the muscle potentiating method due to the inhibition of bloodstream which has been the subject of the present inventor's study.

This muscle potentiating implement is used as wound around a prescribed clamping site of the arm or the leg and consequently enabled to potentiate the muscle of the arm or the leg by inhibiting the bloodstream by the clamping force generated thereby, which implement comprises a belt of a length enough to be wound not less than twice around the perimeter of the clamping site and a hollow gas bag disposed from one end of the belt over a length enough to be wound nearly once around the perimeter of the clamping site and adapted to be filled with a gas.

## Description

### Technical Field

This invention relates to a muscle potentiating implement to be used for potentiating the muscle and more particularly to a muscle potentiating implement which is suitable for carrying out the novel method for muscular potentiation which the inventor in the present patent application has formerly proposed with the object of enabling not only a healthy person but also a person complaining of a disorder in motility to promote muscular potentiation efficiently.

### Background of the Invention

The present inventor has been pursuing a study some time to date with a view to developing a muscle potentiating method which enables the muscular potentiation to be effected esily, safely, and efficiently. As an outcome of this effort, he has filed Japanese patent application, JP-B-05-313,945, and has won grant of Japanese Patent No. 2,670,421.

The method for muscular potentiation according to this patent is based on the following theory. Specifically, the muscular potentiation is generally carried out through a process of "supernormal amelioration" in which the muscle fatigued till fracture of muscular cells assumes, during the course of recovery from the fatigue, a state surpassing the state which existed before the fracture of muscular cells. Though the practice of exerting a load on the muscle as with barbells thereby increasing the kinetic momentum of the muscle is normally adopted for the sake of muscular potentiation, the act of inhibiting the bloodstream in the muscle is likewise capable of giving to the muscle the same kind of fatigue as when the muscle produces an unusually large amount of motion. As a result, the inhibition of the bloodstream can promote the muscular potentiation prominently. The method for muscular potentiation according to the invention described above utilizes the theory of muscular potentiation by the inhibition of bloodstream. To be more specific, this method accompolishes muscular potentiation by a procedure which comprises applying a clamping force for inhibiting bloodstream to the site adjoining the muscle expected to acquire potentiation and falling on the heart's side, namely to the upper site adjoining the muscle, adjusting the clamping force thereby imparting an appropriate load of the inhibition of bloodstream to the muscle, and consequently causing the muscle to develop fatigue and attaining efficient muscular potentiation.

In this method of muscular potentiation, the muscle is efficiently fatigued by inhibiting the bloodstream in the muscle thereby obstructing the supply of oxygen to the muscle and impeding the elimination of such wastes as lactic acid from the muscle. In short, in this method of muscular potentiation, by decreasing the load exerted by a motion on the muscle as compared with that required heretofore and meanwhile giving to the muscle the load due to the inhibition of bloodstream thereby compensating the total amount of the load exerted on the muscle, it is made possible to attain muscular potentiation efficiently.

In this method of muscular potentiation, the fatigue produced in the muscle is copiously increased simply by giving the muscle a light load due to a motion. This method of muscular potentiation, therefore, enjoys outstanding effects such as enabling the target muscle to be specifically potentiated by selecting the position for inhibiting bloodstream and, at the same time, allowing the injury inflicted on the joint and the muscle to be decreased and the duration of pertinent training to be curtailed by decreasing the amount of the actual motion the muscle is required to produce.

The inventor of the subject patent application has continued a study further with a view to widely disseminating the method of muscular potentiation resorting to the inhibition of bloodstream thereby contributing to the promotion of public benefit.

Incidentally, for the performance of this method of muscular potentiation, the use of a muscle potentiating implement which is capable of inhibiting the bloodstream occurring in the muscle expected to attain potentiation and adjusting the clamping force exerted on the muscle while accurately comprehending the clamping force is indispensable.

From this point of view, the present inventor filed Japanese patent application, JP-B-08-248,317, covering an invention relating to a muscule potentiating implement possessed of the functions mentioned above and ultimately won grant of Patent No. 2,796,276.

The muscle potentiating implement to which this patent has issued utilizes a pressure cuff which is widely known as a device for inhibiting the bloodstream occurring in the muscle and adapted to determine blood pressure by effecting inhibition of bloodstream by virtue of the clamping force produced by the pressure of gas.

Specifically, this muscle potentiating implement is composed of a tying belt serving as a part adapted to be put around the muscle and operated to exert pressure on the muscle and having a tube built therein, a pump for supplying a gas to the tube, a gas pressure gauge for measuring the pressure in the tube, and a connecting part for mutually connecting these components, and is provided in the tying belt along the outer lateral face of the tube with a baffle plate.

This baffle plate is provided for the purpose of enabling the clamping force exerted by the tying belt on the arm or the leg to be retained intact even when the tying belt is given a narrower width than in the ordinary pressure cuff so as to avoid obstructing the motion of the muscle by covering the muscle while in use thereon. In short, in the case of the muscle potentiating implement utilizing a pressure cuff, since the tube in the tying belt, when filled with the gas, is inevitably inflated outwardly, the implement possibly encounters a difficulty in retaining to a fully satisfactory extent the clamping force exerted at the site expected to be clamped on the arm or the leg. This trend is particularly prominent when the typing belt is given a decreased width. The muscle potentiating implement mentioned above, therefore, is provided on the outer lateral face of the tube with the baffle plate as laid closely along this face with the object of regulating the direction of the inflation of the tube and causing the tube to be inflated in the direction of clamping the muscle thereby retaining the clamping force on the muscle.

This muscle potentiating implement is veritably excellent in respect that it can be used without any feeling of extraneousness even by an unskilled person because it utilizes the same construction as the pressure cuff familiarized profoundly by persons at large and that it cannot interfere with the motion even when this motion is produced by the muscle kept covered by the implement because the tying belt has a small width.

Even this muscle potentiating implement, however, is not completely devoid of improvement. In case this muscle potentiating implement is put around the arm or the leg and then the pertinent muscle is moved, for example, if the muscle shrinks and consequently grows in diameter, the clamping force exerted on the site for putting the implement around the arm or the leg will possibly grow excessively because the gas in the tube cannot escape outwardly because of the presence of the baffle plate. Further, since the baffle plate is intended to regulate the inflating direction of the tube, it is required to possess a fixed amount of flexibility and a fixed amount of rigidity as well so as to be wound on the arm or the leg. If the arm or the leg is covered in advance with a material possessing such rigidity and then caused to produce a motion, the possibility that the edges of the material possessing the rigidity will cause pain to the arm or the leg is not undeniable. Thus, the desirability of developing a muscle potentiating implement using no baffle plate is believed to find more favorable public acceptance.

The present invention, therefore, has for an object thereof the provision of a muscle potentiating implement which is capable of exerting a fully satisfactory clamping force on the muscle in spite of the absence of a baffle plate and, at the same time, avoiding exertion of any excess clamping force on the site of clamping even when the implement is worn on the site during the production of a motion.

### Disclosure of the Invention

The muscle potentiating implement according to the present invention is used as put around the prescribed site of clamping on the arm or the leg for the purpose of potentiating the muscle of the arm or the leg by inhibiting the bloodstream with the clamping force thereof. It is provided with a belt of a length enough to be wound not less than twice around the perimeter of the clamping site and a hollow gas bag laid from one end of the belt over a length enough to be wound nearly once around the perimeter of the clamping site and adapted to be packed with a gas. This muscle potentiating implement is put to use by having the gas bag wound around the arm or the leg as held in contact with the perimeter of the clamping site and, at the same time, having the excess length of the belt protruding from the gas bag because of the length thereof specified to be not less than twice that of the gas bag wound around the arm or the leg further not less than once from above the gas bag (precisely, from above the belt to which the gas bag is attached).

The muscle potentiating implement of this invention can constantly maintain the clamping force enough for attaining required inhibition of the bloodstream which induces the muscular potentiation because the gas bag is disposed substantially over the entire length of the perimeter of the clamping site and the gas bag, when packed with a gas, is enabled to exert the clamping force in all the directions toward the clamping site. Particularly, this muscle potentiating implement has a construction such that the gas bag is wound nearly once around the clamping site and the excess lengthh of the belt is further wound at least once more on the gas bag. The excess length of the belt, therefore, is eventually caused to regulate the inflating direction of the gas bag toward contacting the arm or the leg and enabled to retain the clamping force amply in spite of the absence of a baffle plate and adapt the muscle potentiating implement for the motion expected to be produced.

Though the belt mentioned above may be formed of such a raw material as thick cloth or leather which is substantially devoid of stretchability, it is preferred to be formed of a raw material possessed of stretchability for the purpose of eliminating the possibility that a motion produced by the muscle will give rise to an excess clamping force. In short, the belt which is possessed of stretchability proves commendable in respect that when the muscle gains in thickness in consequence of a flexing motion of the arm or the leg, the belt itself which has the same function as a baffle plate stretches in the direction of its length and the excess length of the belt mentioned above consequently suffers a decrease in the ability to regulate the gas bag and becomes incapable of exerting an excessive clamping force on the clamping site. For the muscle potentiating method resorting to the inhibition of bloodstream which has been the subject of the present inventor's study, the accurate control of the clamping pressure constitutes itself an essential requirement for the realization of the method. The muscle potentiating implement of this invention, therefore, can promote widespread dissemination of the muscle potentiating method resorting to the inhibition of bloodstream.

Incidentally, since the impartation of flexibility to the gas bag is considered to result in preventing the generation of an excessive clamping force during the production of a motion, the gas bag is also preferred to use a flexible raw material. Particularly, when the belt is formed of a flexible raw material, it is believed necessary that the gas bag be made with such a flexible raw material as is capable of following up the expansion and contraction of the belt. As the gas for filling the gas bag, air is conveniently used. The gas, however, does not need to be discriminated on account of its kind.

Preferably, the muscle potentiating implement according to this invention is provided with fixing means for enabling the belt wound not less than twice around the belt clamping site to be retained intact. The fixing means, for example, may be a fabric fastener which is disposed at the leading terminal of the excess length of the belt. Alternatively, engaging means which utilizes a button or a hook may be adopted as the fixing means.

Incidentally, the length of the belt, besides being enough to be wound not less than twice around the perimeter of the clamping site, must be properly selected to suit the physique of a person determining to attain muscular potentiation, the site of the muscle to be potentiated, and so on and the length of the gas bag must be properly selected to equal roughly the perimeter of the clamping site. The width of the belt and the gas bag may be properly selected in due consideration of the fact that an unduly large width has the possibility of interfering with the production of a motion by covering the muscle excessively and an unduly small width has the possibility of sinking into the muscle and inflicting pain thereto. For use in potentiating the muscle of the arm, the muscle potentiating implement so constructed that the length of the belt is in the range of 70 cm - 130 cm, the length of the gas bag in the range of 20 cm - 50 cm, and the width of the belt and the gas bag in the range of 2 cm - 4 cm is adaptable to almost all persons.
For use in potentiating the muscle of the leg, the muscle potentiating implement so constructed that the length of the belt is in the range of 120 cm - 190 cm, the length of the gas bag in the range of 35 cm - 65 cm, and the width of the belt and the gas bag in the range of 4 cm - 7 cm is adaptable to almost all persons.

### Brief Description of the Drawings

Fig. 1 is a perspective view illustrating a muscle potentiating implement according to the first mode of embodying this invention.
Fig. 2 is a perspective view illustrating the state in which the muscle potentiating implement shown in Fig. 1 is used.
Fig. 3 is a perspective view illustrating the condition in which the muscle potentiating implement according to the second mode of embodying this invention is used.

### Best Mode of Embodying This Invention

Now, the muscle potentiating implement of this invention will be described below with reference to the first and second modes of embodying the present invention. In the following description and drawings, like parts will be denoted by like reference numerals for the purpose of avoiding useless repetitions.

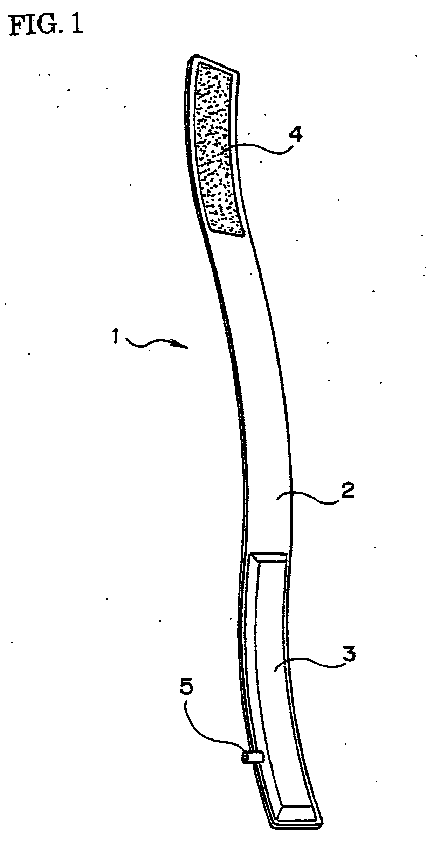

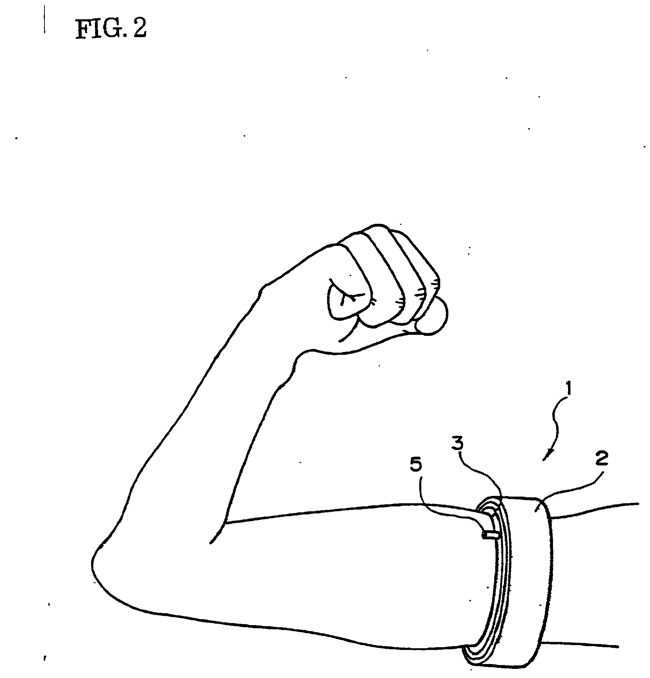

First mode of embodiment: Fig. 1 and Fig. 2 illustrate the first mode of embodiment of a muscle potentiating implement 1. This muscle potentiating implement 1, as is clear from the state of use illustrated in Fig. 2, is intended for the potentiation of the muscle of the arm. This muscle potentiating implement 1 basically comprises a belt 2, a gas bag 3, and fixing means 4.

The belt 2 is formed of Neoprene rubber possessed of flexibility. The raw material for the belt 2 does not need to be limited to Neoprene rubber but may be properly selected from among raw materials possessed of flexibility. The length of this belt 2 has been decided, specifically at 90 cm, in consideration of the fact that the length of the perimeter of the clamping site on the arm of a person engaging in muscular potentiation is 26 cm. This belt 2 has a length enough to be wound not less than twice around the clamping site of the arm with ample allowance. The width of the belt 2 is set at 3 cm.

The gas bag 3 is disposed on one side of the belt 2 in such a manner that the leading terminal part thereof may coincide with one end of the belt. The gas bag 3 is formed of the same rubber possessed of flexibility as that which is used for the rubber bag in the blood pressure gauge. The raw material for the belt 2 does not need to be limited thereto but is only required to be properly selected from among raw materials which are capable of retaining airtightness. The length of the gas bag 3 is nearly equal to the length of the perimeter of the clamping site, i.e. 25 cm, and the width thereof is set at 3 cm. The gas bag 3 is provided with a connecting mouth 5 and is consequently enabled to be connected through the medium of a connecting tube to an air pump (not shown) furnished with a built-in air pressure gauge.

The fixing means 4 is disposed on the side of the belt 2 seating the gas bag 3 over a prescribed range from one terminal part of the belt 2 not seating the gas bag 3. Specifically, this fixing means 4 is formed of a fabric fastener and is so adapted as to be fixed at an arbitrary position on the side of the belt not seating the gas bag 3.

The method for using this muscle potentiating implement will be described below with reference to Fig. 2. First, in preparation for use, this muscle potentiating implement 1 is wound around a clamping site fit for the inhibition of bloodstream necessary for potentiating the muscle and immobilized there. Specifically, the gas bag 3 is wound once around the perimeter of the clamping site and, at the same time, the excess length of the belt 2 is wound further about twice thereon and then the leading terminal part of the belt 2 is fixed with the fixing means. Subsequently, the connecting mouth 5 is connected through the medium of the connecting tube to the air pump (omitted from illustration) and the gas bag 3 is supplied with air by the air pump till the inner pressure thereof reaches a prescribed level. The connecting tube is extracted from the connecting mouth 5 and, at the same time, the connecting mouth 5 is closed with a sealing device (not shown) after the air pressure gauge built in the air pump has confirmed the arrival of the inner pressure of the gas bag 3 at the prescribed level. By allowing the implement to remain in the immobilized state as described above on the muscle or causing the muscle underlying the implement to produce a light motion, the pertinent muscle is potentiated at a speed to a magnitude far exceeding those attainable by the ordinary method of muscle potentiation.

By an experiment of the implement immobilized on the arm in the state described above and caused to produce a flexing motion, it was found that the air pressure which was at 130 mmHg in the absence of the flexing motion rose to 150 mmHg after the start of the flexing motion. The rise of the air pressure on this order fits practical use fully satisfactorily because it has no possibility of causing any problem in terms of safety.

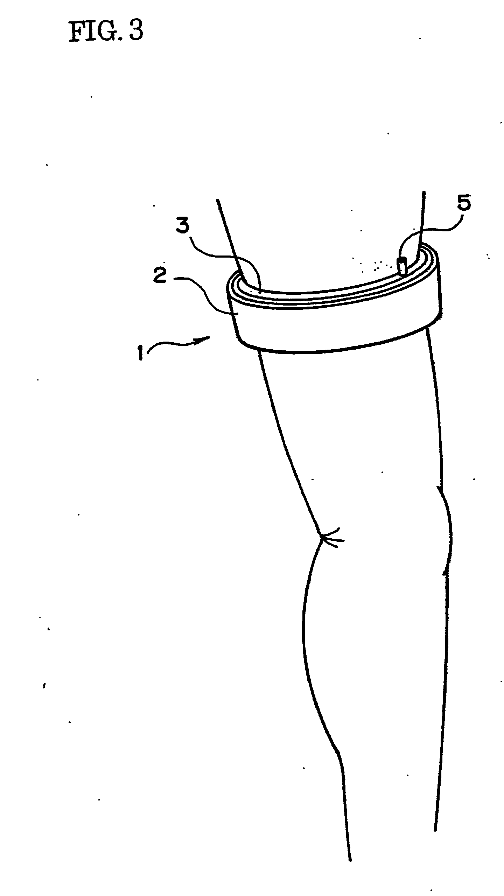

Second mode of embodiment: The muscle potentiating implement 1 according to the second mode of embodiment of this invention will be described below with reference to Fig. 3. This muscle potentiating implement 1 is intended for potentiating the muscle in the leg. It is nearly identical in construction with the muscle potentiating implement 1 according to the first mode of embodiment.

This muscle potentiating implement 1, in consideration of the fact that the perimeter of the clamping site of the leg of an ordinary person using this implement has a length of 45 cm, is so constructed that the length of the gas bag is 45 cm, the length of the belt 2 is 145 cm, and the width of the gas bag 3 and the belt 2 is 5 cm.

Further, since the muscle potentiating implement 1 according to the scond mode of embodiment is formed of a thick cloth, the belt 2 itself is devoid of flexibility. The gas bag 3 of this muscle potentiating implement 1 is likewise devoid of flexibility because it is identical with the gas bag 3 of the first mode of embodiment excepting that it is covered with a bag made of cloth.

The muscle potentiating implement 1 of this mode of embodiment, therefore, is unfit for a person who contemplates wearing the implement on his person and operating it in situ for producing a motion. The method of muscular potentiation resorting to the inhibition of bloodstream described above, however, has high use value for the sake of the rehabilitation of an aged person or a person disabled to produce a motion as by a bodily hurt because it is capable of potentiating the target muscle without requiring impartation of a motion to the muscle.

## Claims

1. A muscle potentiating implement to be used as wound around a prescribed clamping site of the arm or the leg and consequently enabled to potentiate the muscle of the arm or the leg by inhibiting the bloodstream by the clamping force generated thereby, which comprises a belt of a length enough to be wound not less than twice around the perimeter of the clamping site and a hallow gas bag disposed from one end of the belt over a length enough to be wound nearly once around the perimeter of the clamping site and adapted to be filled with a gas.

2. An implement according to claim 1, wherein said belt is formed of a raw material possessed of flexibility

3. An implement according to claim 1 or claim 2, wherein said gas bag is formed of a raw material possessed of flexibility.

4. An implement according to any of claims 1 - 3, wherein said belt is provided with fixing means for retaining in tact the belt held as wound around the clamping site.

5. An implement according to any of claims 1 - 4, wherein the length of said belt is set at 70 cm - 130 cm, the length of said gas bag at 20 cm - 50 cm, and the width of said belt and said gas bag at 2 - 4 cm.

6. An implement according to any of claims 1 - 4, wherein the length of said belt is set at 120 cm - 190 cm, the length of said gas bag at 35 cm - 65 cm, and the width of said belt and said gas bag at 4 cm - 7 cm.
